# EUROPEAN PATENT APPLICATION

(11) **EP 2 343 081 A1**
(43) Date of publication of application: **13.07.2011**
(21) Application number: 09181049.9
(22) Date of filing: 31.12.2009
(51) Int. Cl.: A61K 38/12, A61K 38/21, C07K 5/00, C07K 14/57, A61P 37/00, A61P 35/00, A61P 31/12, A61P 43/00, A61P 1/16, A61P 9/10, A61P 11/00

(54) **Interferon analogs**

(71) Applicant: Rijksuniversiteit Groningen, 9712 CP Groningen (NL); Stichting voor de Technische Wetenschappen, 3527 JP Utrecht (NL)
(72) Inventor: The designation of the inventor has not yet been filed
(74) Representative: Hatzmann, Martin

(57) **Abstract**

The invention relates to the field of medicine. Among others, it relates to biologically active analogs of interferons (IFNs) which show less unwanted side-effects and to the therapeutic uses thereof. Provided is an IFN analog, wherein the moiety mediating binding to its natural receptor is at least functionally disrupted and wherein the analog comprises a signaling moiety capable of mediating intracellular IFN activity, said signaling moiety being provided at its N-terminus, optionally via a linker, with at least one targeting domain capable of binding to a cell surface receptor other than the IFN receptor.

## Description

The invention relates to the field of medicine. Among others, it relates to biologically active analogs of interferons (IFNs) which show less unwanted side-effects and to the therapeutic uses thereof.

About ten distinct IFNs have been identified in mammals; seven of these have been described for humans. They are typically divided among three IFN classes: Type I IFN, Type II IFN, and Type III IFN. All type I IFNs bind to a specific cell surface receptor complex known as the IFN-α receptor (IFNAR) that consists of IFNAR1 and IFNAR2 chains. The type I interferons present in humans are IFN-α, IFN-β and IFN-ω. Interferon type II binds to IFNGR. In humans this is IFN-γ. By interacting with their specific cell surface receptors, IFNs activate signal transducer and activator of transcription (STAT) complexes; STATs are a family of transcription factors that regulate the expression of certain immune system genes. Some STATs are activated by both type I and type II IFNs. However each IFN type can also activate unique STATs (Platanias, L. C. 2005 Nature reviews. Immunology 5 (5): 375-386).

IFNs belonging to all IFN classes are very important for fighting viral infections. Although they are named after their ability to "interfere" with viral replication within host cells, IFNs have other functions: they activate immune cells, such as natural killer cells and macrophages; they increase recognition of infection or tumor cells by up-regulating antigen presentation to T lymphocytes; and they increase the ability of uninfected host cells to resist new infection by virus. Certain host symptoms, such as aching muscles and fever, are related to the production of IFNs during infection.

IFNγ is a pleiotropic cytokine produced by the activated immune cells. It acts through the IFNγ receptor that is expressed nearly on all cell types, however it displays a strict species specificity. IFNγ has been applied for the treatment of immunological, viral and cancer diseases (Younes and Amsden, J Pharm Sci 2002) with significant effects. In addition, several studies have demonstrated the potential role of IFNγ in renal and liver fibrosis (Kidney Int. 1999 ;56:2116-27, Hepatology 1996 23:1189-99).

Unfortunately, the short circulation half-life and undesirable systemic side effects of currently available interferons have limited its clinical application and even halted clinical trials. Many attempts have been made to circumvent these problems e.g. by incorporating IFNγ into liposomes, microspheres and elastomers (Pharm Res. 2000 17 :42-8, Pharm Res. 1996 13:1464-75, J Control Release. 2005 102:607-17). Cell-specific delivery approaches have not been used so far. This is not surprising in view of the fact that such an approach is generally deemed impossible for cytokines which need to be delivered to their own receptors to elicit a biological effect, so that they always end up in target cells that express these receptors. Delivery to other target receptors will therefore in most cases be useless and at best lead to uptake in other cell types causing loss of activity or further diversification of adverse effects.

However, the present inventors recognized that the structure of INF offers unique targeting opportunities since the molecule contains a receptor binding moiety which is species-specific and a signalling moiety which is non-species specific and which acts intracellularly. It was surprisingly found that that the unique structure of INFγ allows delivery of the signalling moiety of IFNγ to another target receptor, provided that this new target receptor allows intracellular release of this signalling moiety and subsequent activation of the intracellular/nuclear INFγ signalling pathway. For example, a truncated IFNγ mimetic targeted to the Platelet Derived Growth Factor (PDGF) receptor displayed significantly less systemic side effects in an acute liver injury mouse model when compared to full length IFNγ or non-targeted IFNγ mimetic.

Accordingly, the invention relates to an analog of interferon (IFN), wherein the moiety mediating binding to its natural receptor is at least functionally disrupted and wherein the analog comprises a signaling moiety capable of mediating intracellular IFN activity, said signaling moiety being provided at its N-terminus, optionally via a linker, with at least one targeting domain capable of binding to a cell surface receptor other than the IFN receptor. Preferably, the analog is an IFN gamma (IFNγ) analog.

Interferon analogs provided with a heterologous targeting moiety are known in the art. WO2008/068621 discloses a conjugation product of IFNγ and a targeting moiety, such as a tumor targeting moiety or a tumor vasculature targeting moiety. The prior art only discloses conjugation to full length IFNγ, which will still bind to its native receptor causing adverse effects.

EP0844252 aims to provide cyclic peptides and their preparation process, which allow subsequent chemical grafting on coupling on said cyclic peptides, i.e. their attachment to a solid support, to a high molecular weight compound, to a marker and/or to other cyclic peptides, in order to provide new or improved biotechnological applications, particularly in the field of affinity chromatography, immunisation, development of diagnostic tests, vaccines and pharmaceutical compositions. It teaches the coupling of cyclic peptide ligands to biological molecules, among others to interferons. No mention is made in EP0844252 about the use of a truncated IFN molecule lacking a functional binding domain to its natural receptor.

An interferon analog of the present invention has clear and unexpected advantages over the use of a (modified) full length interferon, not only with respect to its therapeutic use (less side-effects, increased efficacy, reduced antigenicity because of lower molecular weight) but also to its production process (recombinant synthesis).

The functional disruption of the moiety mediating binding to its natural receptor can be achieved by deleting, inserting, substituting one or more relevant amino acid residues in the receptor binding domain. Interferon receptor binding domains have been identified and are known in the art. For example, binding of murine IFNγ to the INFγ receptor can be abolished by at least partially deleting the first 40, 30 or 20 N-terminal residues.

In one embodiment, the analog is a truncated IFNγ polypeptide which only contains the residues involved in intracellular signalling. As used herein, intracellular signalling may comprise nuclear translocation and/or anti-viral activity.

Preferably, the signaling moiety mediating intracellular activity comprises a polybasic nuclear localization signal (NLS) motif as found in the C-terminus of human and murine IFN (Subramaniam et al. 2000, J. Cell Science 113, 2771-2781). This NLS motif is thought to form a complex with a signal transducer and activator of transcription (STAT); STATs are a family of transcription factors that regulate the expression of certain immune system genes. Some STATs are activated by both type I and type II IFNs. However each IFN type can also activate unique STATs (Platanias, L. C. 2005 Nature reviews. Immunology 5 (5): 375-386) STAT activation initiates the most well defined cell signaling pathway for all IFNs, the classical Janus kinase-STAT (JAK-STAT) signaling pathway.In this pathway, JAKs associate with IFN receptors and, following receptor engagement with IFN, phosphorylate both STAT1 and STAT2. As a result, an IFN-stimulated gene factor 3 (ISGF3) complex forms - this contains STAT1, STAT2 and a third transcription factor called IRF9 - and moves into the cell nucleus. Inside the nucleus, the ISGF3 complex binds to specific nucleotide sequences called IFN-stimulated response elements (ISREs) in the promoters of certain genes; this induces transcription of those genes.

An analog provided herein may comprise the polybasic NLS motif comprises the amino acid sequence (R)KRXRS(R), wherein X is any amino acid residue, preferably wherein X is R, K, S or T. Preferably, the NLS motif is present at the C-terminal end of the analog. In one embodiment, it comprises the sequence, preferably the C-terminal sequence RKRKRSR, KSKRSR, KRTRS or KRTRSQ. In a specific aspect, the signaling moiety comprises or consists of a sequence selected from the group consisting of
(a) the amino acid sequence KFEVNNPQVQRQAFNELIRVVHQLLPESSLRKRKRSR;
(b) the amino acid sequence YSVTDLNVQRKAIHELIQVMAELSPAAKTGKRTRSQ
(c) a stretch of at least 10, preferably at least 15, contiguous amino acids of the sequence of (a) or (b);
(d) the consensus sequence VxxxxVQRxAxxELIxVxxxLxPxxxxxKRxRS wherein x is any amino acid residue;
(e) an amino acid sequence showing at least 70%, preferably at least 80%, more preferably at least 90% identity to a) or b) provided that the intracellular signaling activity is maintained; and
(f) the amino acid sequence under (a) or (b) wherein at most 10, preferably at most 8, more preferably at most 5 amino acid residues are deleted, added or substituted, provided that the signaling activity, for example nuclear translocation, is maintained.

The amino acid sequence under (a) represents a truncated murine INFγ sequence, wherein the sequence under (b) is a human homologue. Preferably, an analog of the invention comprises a stretch of at least 10, preferably at least 15, more preferably at least 20 contiguous amino acids of the sequence of (a) or (b). In one embodiment, said stretch comprises the N-terminal sequence of the sequence under (a) or (b). In another embodiment, it comprises the C- terminal sequence of the sequence under (a) or (b). In yet another embodiment, the stretch comprises an internal sequence of the sequence under (a) or (b). Exemplary sequences are LLPESSLRKRKRSR, KFEVNNPQVQRQ, QAFNELIRVVHQLL, MAELSPAAKTGKRTRSQ, YSVTDLNVQRKAI, KAIHELIQVMAELS.

The skilled person will understand that variants with one or more amino acid modifications to the sequences under (a) or (b) are also within the scope of the invention. the amino acid sequence under (a) or (b) wherein at most 10, preferably at most 8, more preferably at most 5 amino acid residues are deleted, added or substituted, provided that the signaling activity, for example nuclear translocation, is maintained.

Alignment of the human and murine INFγ sequences shows that 15 out of the 36 residues (41%) are identical and 24 out of the 36 residues (66%) are positively charged. The identity match is done according to clustal W alignment software.

huInterferon gamma 4 YSVTDLN VQR K AIHE L I Q V MAELS PAAKTG KRTRSQ 39
+ V + VQR + A + E L I + V + +L P + KR RS+
MuInterferon gamma 3 FEVNNPQ VQRQ AFNE L I RV VH QLL PESS LRKRK RS R 38

In one embodiment, an analog comprises a signalling moiety according to the consensus sequence (d) mentioned above. Other useful sequences include an amino acid sequences showing at least 70%, preferably at least 80%, more preferably at least 90%, most preferably at least 95% identity to (a) or (b) provided that the intracellular signaling activity is maintained.

Preferably, an analog comprises a sequence corresponding to residues 95-133 in murine IFNγ or residues 95-134 in human IFNγ. This sequence has antiviral activity (Mujtaba et al. 2006, Clinical and Vaccine Immunology, Vol. 13, No.8, p.944-952).

The signaling activity of an analog can be readily determined by methods known in the art. For example, the capacity of the analog to induce nuclear translocation of STAT1α when taken up intracellularly by a murine macrophage cell line can be determined as described by Subramaniam et al. Signaling activity can also be assessed by complex formation with the transcription factor Statlalpha and the nuclear importer of Statlalpha, the importin-alpha analog NPI-1. See in particular Subramaniam et al. (2001, J. Interferon Cytokine Res. 21(11):951-959). Other suitable *in vitro* assays include NO-production in murine macrophages such as the RAW cell line. In one embodiment, the analog shows at least 30%, preferably at least 50%, more preferably at least 75% of the (in vitro) activity of its full length interferon counterpart. Cell specific targeting can significantly enhance the *in vivo* efficacy of an analog showing relatively low activity *in vitro.*

As mentioned herein above, an analog of the present invention is characterized by the absence of a functional IFN-receptor binding domain and the presence of a targeting domain capable of binding to an alternative receptor which can mediate intracellular uptake of the analog, for example a receptor which enters the endocytic pathway upon ligand binding and/or as part of constant receptor turnover. As will be understood, the alternative or "secondary" receptor to be targeted should have some degree of cell type specific expression. Ubiquitously expressed receptors are less suitable candidates. Furthermore, the receptor must be expressed on a cell which is responsive to the intracellular signaling moiety, e.g. in case of an IFNγ analog it must contain an IFNγ-responsive element.

Preferably, the targeting domain can bind to a receptor that is specific for fibroblast and fibroblast-like cells, such as for example myofibroblasts, portal fibroblasts, mesangial cells, interstitial fibroblasts, alveolar fibroblasts or stromal cells. Also envisaged are receptors expressed on tumor cells. An IFNγ - analog can induce apoptosis in tumor cells, which can be specifically targeted via a secondary receptor expressed on tumor cells. In one embodiment, the receptor is selected from the group consisting of the PDGF receptors, collagen type VI receptor, cytokine receptors including TGFβ receptor, TNFα receptor and the IL1β receptor, Insulin growth factor receptors, VEGF receptors and chemokine receptors (e.g. CXCR4). Very good results were obtained when the analog was targeted to the PDGF receptor, preferably the PDGF-β receptor.

The targeting domain is a preferably a proteinaceous substance (peptide) such that the analog as a whole is a fusion polypeptide which can be readily produced e.g. recombinantly. However, non-proteinaceous other types of targeting domains are also envisaged such as saccharides, lipids, nucleic acids, synthetic molecules, and the like. For example, mannose-6-phosphate (M6P) or derivative thereof can be used as targeting domain for the M6P/IGF II receptor. See for example EP1117443.

In one aspect, the targeting domain comprises at least one cyclic peptide portion. Peptides can be cyclised by various means, including cysteine-disulfide or lanthionine bridge formation. Cyclic peptides useful for targeting an analog of the invention to a desired cell type are described in the art. For example, EP1117443 discloses a cyclic peptide comprising at least one sequence encoding a cell receptor recognising peptide (RPR). In one embodiment, an analog of the invention comprises in its (cyclised or linear) targeting domain at least an amino acid sequence selected from the group consisting of RGD, KPT, SRN, NLI and LID.

A preferred embodiment relates to analogs having a targeting domain which comprises multiple receptor binding sequences, preferably in the form of at least one tandem repeat of a (cyclic) peptide portion. The tandem repeat may comprise two cyclic peptide portions, preferably identical cyclic peptide portions, connected via a linker of 1 to 5 amino acids. This is of particular advantage for targeting a receptor which is active as a dimer, such as the PDGF, TGFβ or IL-10 receptor.

In one embodiment, the targeting domain comprises one, preferably two, copie(s) of the amino acid sequence X₁SRNLIDX₂, wherein X₁ and X₂ denote moieties which together can form a (peptidic) bond such that a cyclic structure is formed wherein the sequence SRNLID is part of the ring. The two copies are preferably spaced by a linker sequence of 1 to 7 amino acids. Preferably, X₁ and X₂ are Cys residues. For instance, the inventors found that a targeting domain comprising the amino acid sequence CSRNLIDC-linker- CSRNLIDCS, wherein the linker is an amino acid sequence of 1 to 7, preferably 4 or 5, amino acid residues, is very effective in binding to the dimeric PDGFβ receptor. The linker may consist of 4 or 5 amino acid residue, preferably selected from the group of Gly, Ala, Ser and Thr residues, more preferably at least 3 of them being a glycine residue. As a specific example, the targeting domain comprises or consists of the amino acid sequence CSRNLIDCKGSGGCSRNLIDCS.

The targeting domain may be attached to the signalling domain by any means, for instance by a linker or spacer sequence. Suitable linker sequences are typically up to 15, preferably up to 10 amino acid residues in length. Polyalanine linkers may be used. For instance, provided herein is an interferon gamma analog, consisting of the sequence CSRNLIDCKGSGGCSRNLIDCS AAA AKFEVNNPQVQRQAFNELIRVVHQLLPESSLRKRKRSR

The invention also relates to a conjugate comprising a compound of interest, e.g. a biologically active molecule, conjugated to a bicyclic PDGF-receptor-targeting domain, said targeting domain comprising two copies of the amino acid sequence X₁SRNLIDX₂, wherein X₁ and X₂ denote moieties which together can form a (peptidic) bond such that a bicyclic structure is formed wherein the sequence SRNLID is part of each ring. Preferably, X₁ and X₂ are Cys residues. The two copies are spaced by a linker sequence of 2 to 7 amino acids. It was found that this spacer length allows for highly efficient targeting of the PDGF receptor, which is active as a dimer. Thus, also provided is a conjugate comprising a compound of interest conjugated to a targeting domain, said targeting domain comprising the amino acid sequence X₁SRNLIDX₂-linker- X₃SRNLIDX₄, wherein the pair of X₁ and X₂ and the pair of X₃ and X₄ can form a (peptidic) bond such that a bicyclic structure is formed wherein the sequences SRNLID are part of a ring, and wherein the linker is an amino acid sequence of 2 to 7, amino acid residues.

Very good results were achieved with a linker of 3 to 5 amino acid residues. In one embodiment, the conjugate comprises the amino acid sequence CSRNLIDC-linker- CSRNLIDCS (BiPPB) wherein the linker is an amino acid sequence of 2 to 7, preferably 3 to 5, amino acid residues. This bicyclic peptide is suitably used as low molecular weight targeting ligand with high affinity to the PDGF receptor (PDGF-R). The peptide can be prepared either by chemical or recombinant synthesis. The linker may comprise one or more amino acid residues with a reactive side chain that can be used for covalent attachment of a compound of interest, like a detectable label, a drug and/or diagnostic. Suitable reactive amino acids include lysine, serine and threonine, arginine, histidine, aspartic acid, glutamic acid, cysteine, asparagine, glutamine, tyrosine, methionine and tryptophan.

The biologically active moiety may have any type of useful activity, including cytokine, chemokine, or prostaglandin activity. It can be of proteinaceous or non-proteinaceous nature. For instance, the moiety is selected from the group selected from drugs, cytokines, chemokines, hormones, prostaglandins, and the like. Specific examples include PGE2, 15d-PGJ2, IL-10, IFNγ, truncated IFNγ. Proteinaceous moieties may conveniently be attached to the PDGF-R specific targeting domain by genetic fusion, at either the N- or C-terminus. In one embodiment, it is conjugated to the N-terminus.

An analog or conjugate according to the invention may be coupled to a core and/or carrier or delivery molecule by methods known in the art. Suitable cores or carriers include dendrimers, liposomes, and natural, synthetic or semi-synthetic polymers (branched or linear). Dendrimers have successfully proved themselves as useful additives in different routes of drug administration because they can render drugs greater water-solubility, bioavailability, and biocompatibility. See Chen et al., Journal of Pharmaceutical Sciences, Vol. 97 Issue 1, pg. 123 - 143. As an example, the invention provides an IFNy-analog conjugated to a dendrimer or to a liposome. The liposome may contain an (anti-cancer) drug.

In one embodiment, an interferon analog or PDGFR-targeted conjugate according to the invention is modified by conventional means to improve its pharmacological properties, like enhancing the efficacy and/or stability. In one embodiment, it is modified in order to enhance the half life by the attachment of at least one non antigenic polymer, for instance by a polymer selected from the group consisting of polyethylene glycols (PEGs) and derivatives thereof. PEGylation is routinely achieved by incubation of a reactive derivative of PEG with the target macromolecule. The covalent attachment of PEG to a drug or therapeutic protein can "mask" the agent from the host's immune system (reduced immunogenicity and antigenicity), increase the hydrodynamic size (size in solution) of the agent which prolongs its circulatory time by reducing renal clearance.

A further aspect of the invention relates to an isolated nucleic acid sequence encoding a proteinaceous interferon analog according to the invention or encoding a proteinaceous PDGFR-targeted conjugate as described herein above. The skilled person will be able to design and construct a suitable nucleic acid sequence e.g. a fusion construct that encodes both the targeting moiety and the biologically active moiety using standard recombinant DNA technology. The isolated nucleic acid sequence may be part of an expression vector, for example a vector designed for recombinant protein production in a bacterial or mammalian host cell. Also provided is a host cell comprising a nucleic acid sequence or a vector according to the invention, preferably wherein said host cell is a bacterial or mammalian host cell.

An analog or PDGF-targeted conjugate disclosed herein has improved properties with respect to its distribution within the body. More specifically, it allows directing a biologically active compound of interest to a cell of interest while maintaining the biological activity of that particular compound. To attain cell-specificity, these mediators are equipped with an address label that will increase their concentration around relevant target receptors in diseased tissue. A further embodiment therefore relates to a pharmaceutical composition comprising an IFN analog or a PDGF-targeted conjugate and a pharmaceutically acceptable carrier. A specific aspect relates to a pharmaceutical composition comprising a targeted IFNγ analog, preferably a PDGF-R-targeted IFNγ analog showing reduced side-effects as compared to non-targeted IFNγ. An exemplary pharmaceutical composition contains a peptide comprising or consisting of the sequence CSRNLIDCKGSGGCSRNLIDCSAAA AKFEVNNPQ VQRQAFNELIRVVHQLLPESSLRKRKRSR. Another exemplary pharmaceutical composition comprises a conjugate comprising a biologically active molecule conjugated to a targeting domain, said targeting domain comprising the amino acid sequence CSRNLIDC-linker- CSRNLIDCS, wherein the linker is an amino acid sequence of 2 to 7, preferably 3 to 5, amino acid residues.

Also provided is the use of an IFN(γ) analog according to the invention for the manufacture of a medicament for the therapeutic or prophylactic treatment of a disease selected from cancer, viral diseases, fibrotic disease, sclerotic disease and chronic or acute inflammatory processes such as glomerulosclerosis, interstitial fibrosis, lung fibrosis, atherosclerosis, rheumatoid arthritis, Crohns disease, colitis ulcerosa, glomerulonephritis and sepsis.

The invention also relates to a method for therapeutic or prophylactic treatment of a disease selected from cancer, viral disease, fibrotic disease, sclerotic disease and chronic or acute inflammatory processes such as glomerulosclerosis, interstitial fibrosis, lung fibrosis, atherosclerosis, rheumatoid arthritis, Crohns disease, colitis ulcerosa, glomerulonephritis and sepsis, comprising providing to a subject in need thereof a therapeutically effective dose of an IFN analog according to the invention. The method may be a liver disease, preferably a chronic liver disease such as liver cirrhosis. The person skilled in the art will adjust the dosage to be applied to the manner of application, size, weight, state of health etc of the subject to which administration is to occur. Administration can occur in any manner known per se for administration of a medicament.

### LEGEND TO THE FIGURES

Figure 1: cloning of mimetic IFNγ from the mouse. Splenocytes were stimulated with PHA and RNA was isolated. RT and PCR using specific primers yielded a PCR product of the expected size. Expression of the construct in BL21 cells and Western blotting confirmed production of the mimetic.

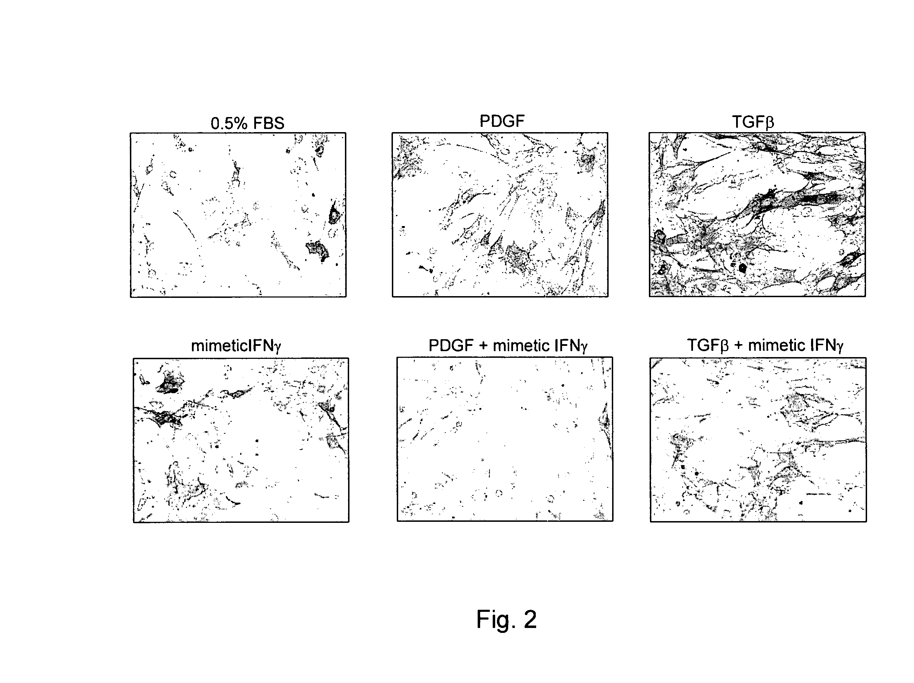

Figure 2: Recombinant mimetic IFNγ shows anti-fibrotic effects in mouse 3T3 fibroblasts. Cells are stained for α-Smooth muscle actin which is a fibrosis marker.
Figure 3: Construction of pET39b-BiPPB encoding a bicyclic PDGF-receptor targeting domain.
Figure 4: Construction of pET39b-BiPPB-IFNgamma encoding an PDGFR-targeted IFNγ conjugate.
Figure 5: Construction of pET39b-BiPPB-mimeticIFNγ.
Figure 6: Dot Blot analysis of IFNγ-BiPPB and Mimetic IFNγ-BiPPB showing the coupling of pPB-peptide to INFγ.
Figure 7: Binding study in human hepatic stellate cell line LX2 cells. Cells were stained for PDGFR-binding peptide. Staining indicates the binding of pPB-containing constructs to the target cells.
Figure 8: Blood count analysis to study side effects of the treatment in acute liver injury mouse model. WBC= white blood cell count. LYM= lymphocytes. PLT= platelet count. RBC= red blood cell count. For details see Example 4.
Figure 9: Real Time PCR analysis of the liver fibrosis markers MMP13 and TIMP1. For details see Example 4.

### EXPERIMENTAL SECTION

Liver Fibrosis is characterized by the excessive accumulation of the extracellular matrix components that leads to hepatic scars. To date, no successful therapy is available for the treatment of liver fibrosis. Hepatic stellate cells (HSCs) and fibroblasts are the key effector cells involved in the progression of the disease, which are activated by crucial growth factors like Platelet derived growth factor (PDGF) and transforming growth factor-beta (TGFβ). Interferon gamma (IFNγ) has been shown to have various beneficial effects *in-vitro* and *in-vivo* during liver fibrosis. However, IFNγ displays a strict species specificity and has a short circulating half life whichlimits its potential clinical use. Moreover, INFγ has serious adverse effects on the immune system, on endothelial cells and on the Central Nervous System (e.g. causing depressions) that all lead to frequent withdrawal of patients from clinical trials and consequently to failure of these trails. To circumvent these drawbacks, a stable peptide mimetic of IFNγ has been produced that lacks the extracellular receptor recognition site and contains a signalling moiety which interacts directly at the downstream IFNγ signalling cascade thereby retaining the prospective functions of IFNγ.

To increase the specificity of IFNγ and IFNγ mimetic peptide, IFNγ and mimetic peptide fused to BiPPB (**Bi**cyclic **p**eptide against the **P**DGF-**b**eta -receptor) have been generated, since PDGF receptor expression is highly up-regulated during liver injury particularly in HSCs.

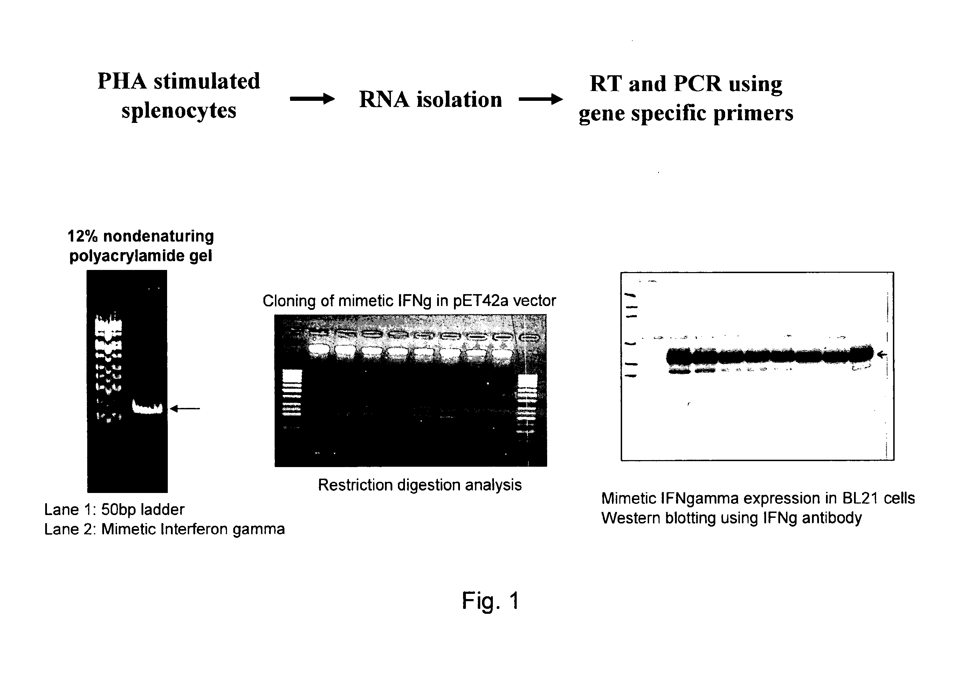

**Example 1: Cloning of mimetic IFN**γ Mouse splenocytes were isolated from fresh spleens and were cultured in the presence of PHA (Phytohemagglutinin) to stimulate cytokine production. After 24 hrs of stimulation, RNA was isolated and cDNA was synthesised using gene specific reverse primer followed by PCR amplification by phusion DNA polymerase using mimetic IFNγ specific forward and reverse primers. The obtained fragment was cloned in pET42a (prokaryotic expression vector) at PshA1/EcoRI site and the positive clones were checked by restriction digestion analysis. See Figure 1.

The 5'-> 3' nucleotide sequence of the truncated mouse Interferon gamma (NCBI Reference sequence: NM_008337.2) (nt 457 - nt 572) is as follows:

457 gcca agtttgaggt caacaaccca caggtccagc gccaagcatt caatgagctc atccgagtgg tccaccagct
gttgccggaa tccagcctca ggaagcggaa aaggagtcgc tg 572 a

The last nucleotide has been added in order to insert the stop codon before cysteine, which is the last amino acid in the sequence. Cysteine is removed from the sequence to provide appropriate folding of the peptide and also to avoid inappropriate folding due to disulfide bonds in the fusion protein (with BiPPB).

The encoded amino acid sequence is
AKFEVNNPQVQRQAFNELIRVVHQLLPESSLRKRKRSR*

* Denotes Stop codon

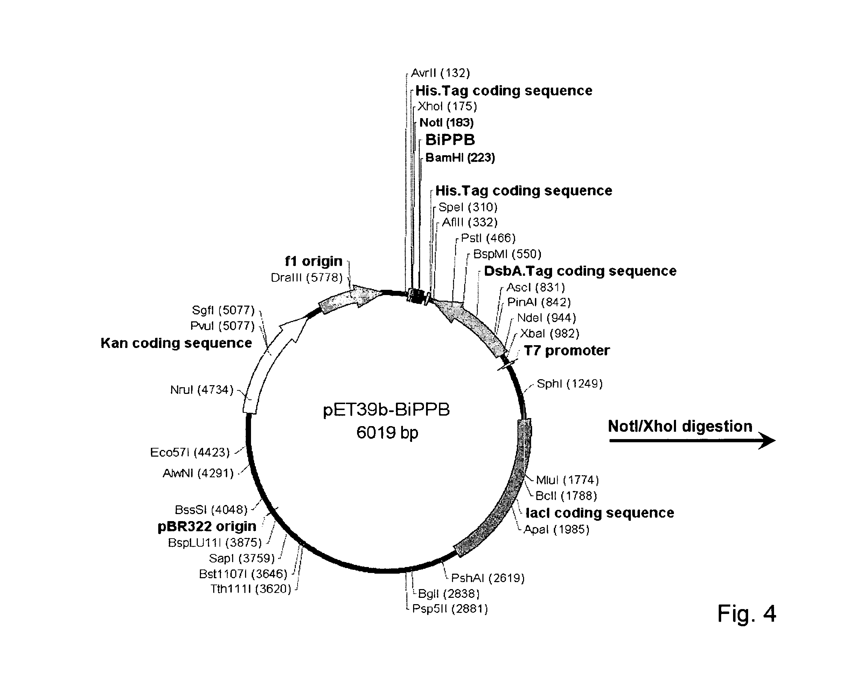

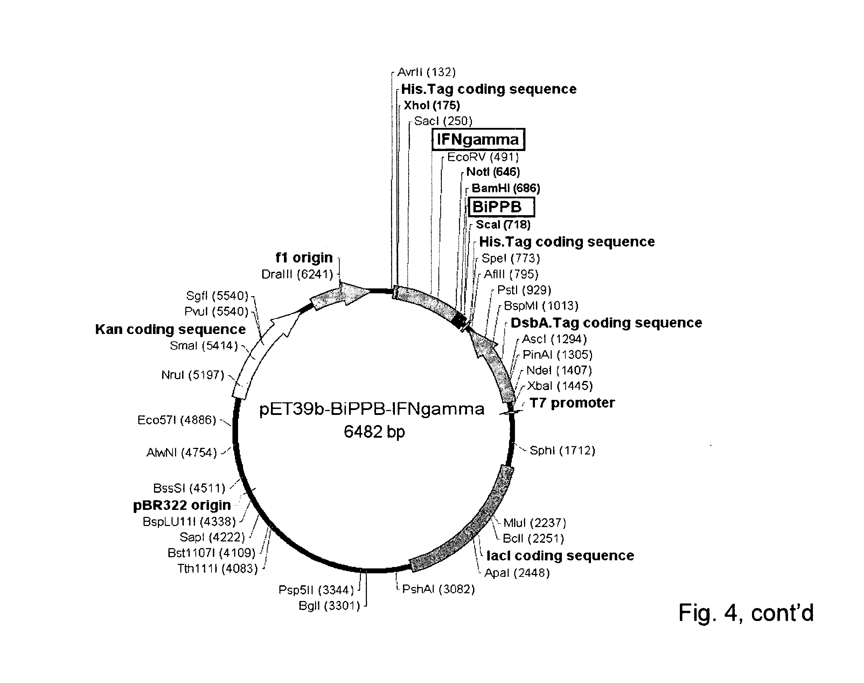

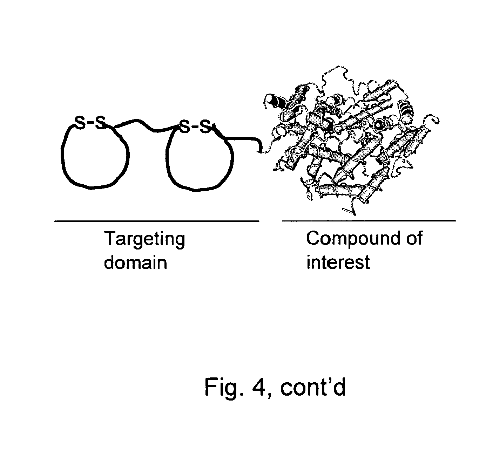

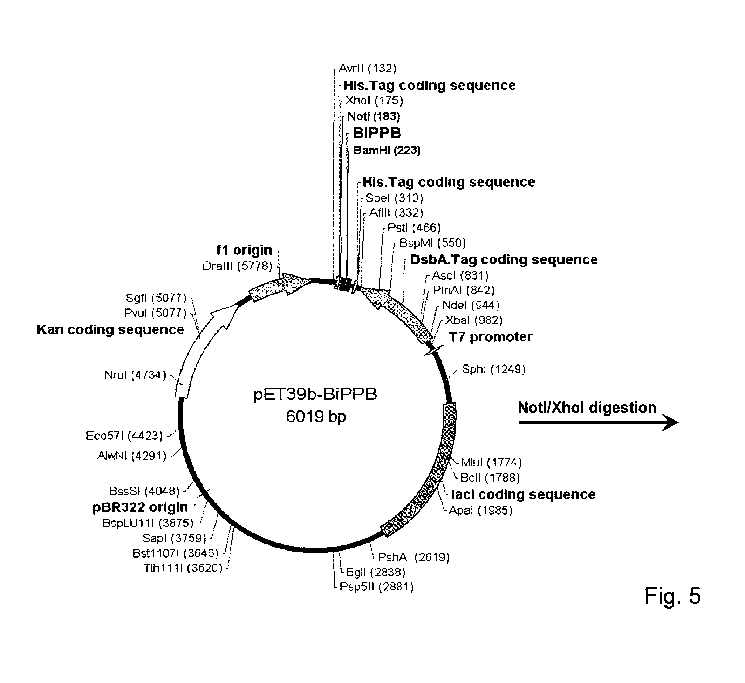

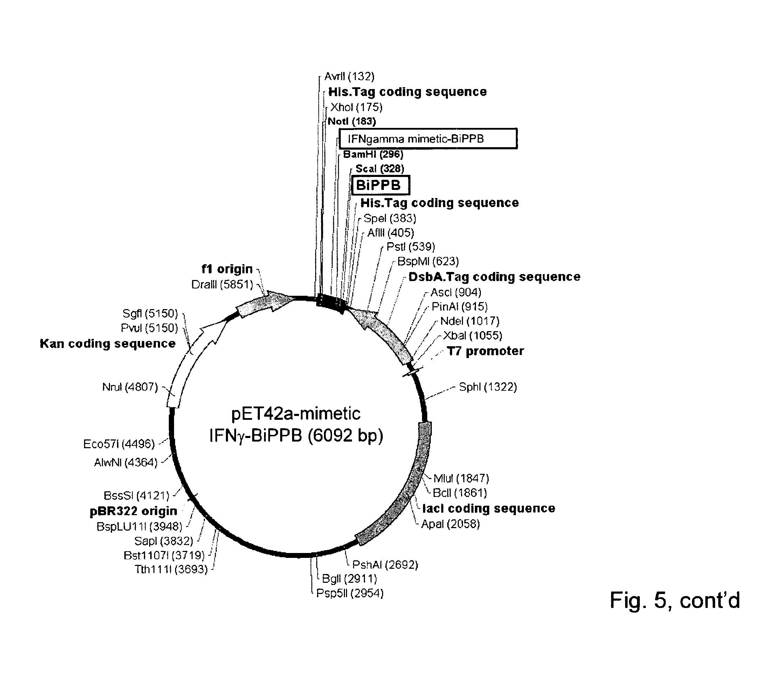

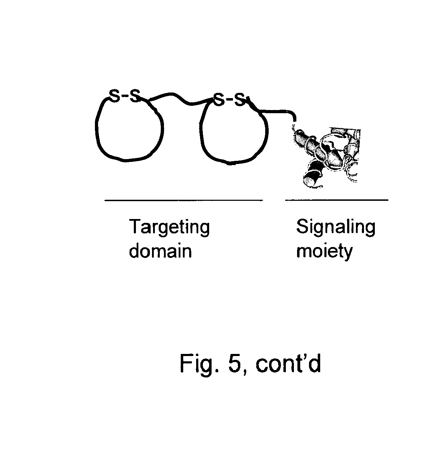

**Cloning of IFNγ-BiPPB and mimetic IFN**γ**-BiPPB** A nucleic acid sequence encoding the bicyclic PDGF targeting domain BiPPB was generated by amplification of two fragments using 4 primers (2 for each fragment) and then ligated using inbuilt Bam HI restriction site and was then cloned in pET39b vector at ScaI/NotI site. IFNγ and Mimetic IFNγ was PCR amplified using peptide fusion primer (forward) and IFNγ or mimetic IFNγ reverse primer. The amplified fragment was digested and ligated in pET39b-BiPPB vector at NotI/XhoI site and the positive clones were checked by restriction digestion analysis. See Figures 4 and 5. The sequences of all the constructs were further confirmed by automated DNA sequencing.

### BiPPB

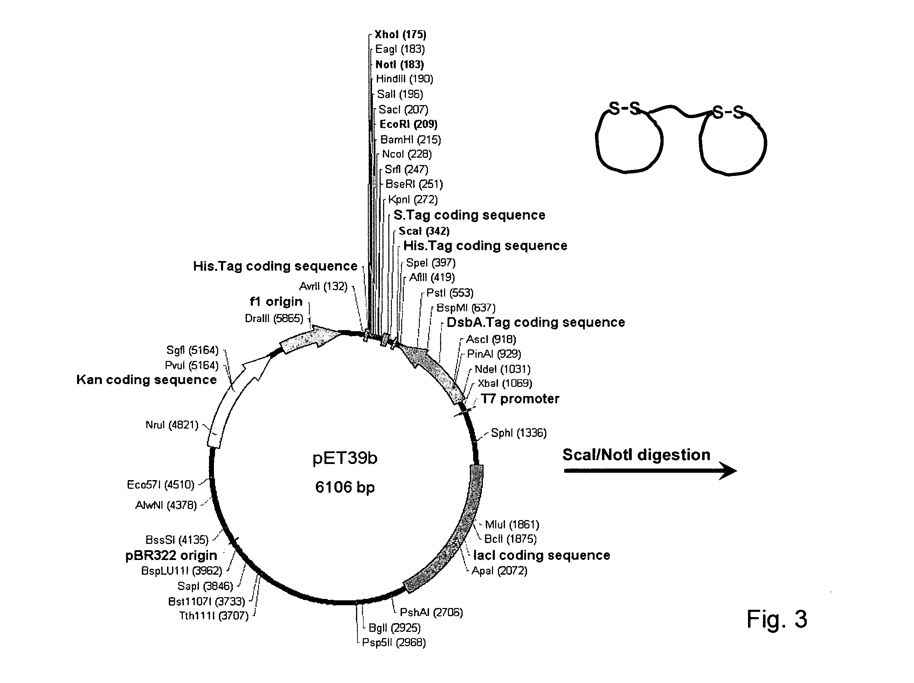

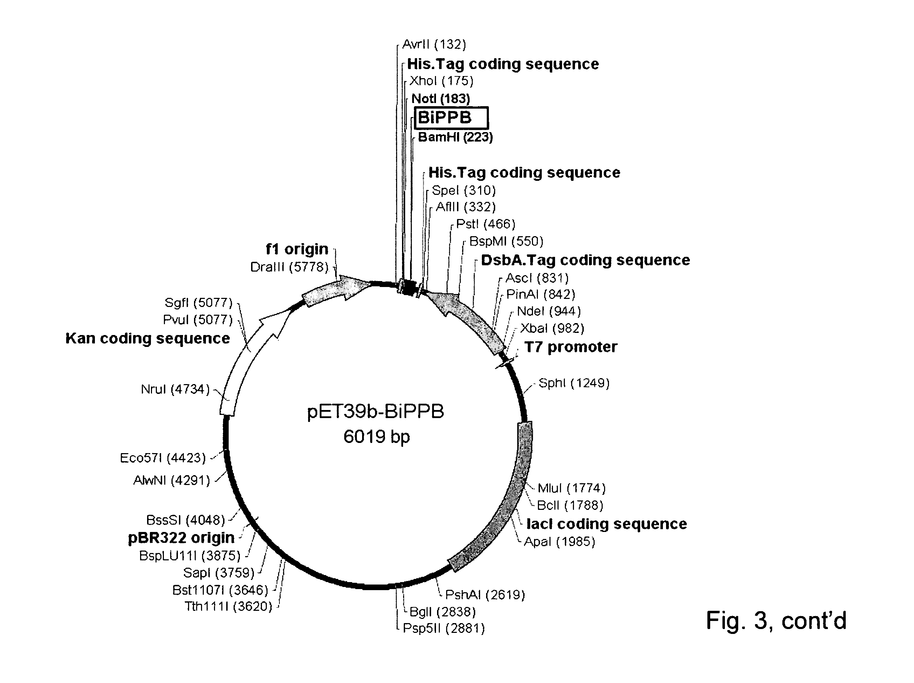

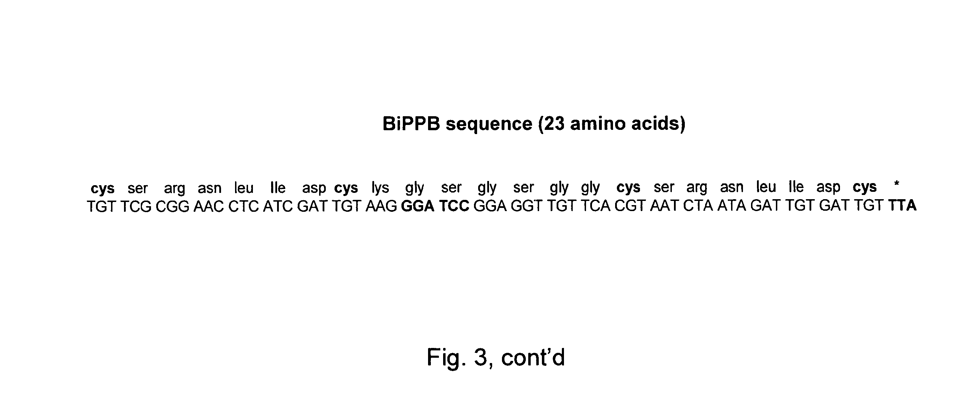

Nucleotide sequence for BiPPB:
tgt tct aga aac ctc atc gat tgt aag gga tcc gga ggt tgt tca cgt aat cta ata gat tgt tca Amino acid sequence for BiPPB: CSRNLIDCKGSGGCSRNLIDCS (see also Figure 3)

### Interferon gamma (full length)

Nucleotide sequence: mouse Interferon gamma (NCBI Reference sequence: NM_008337.3)

gcggccgca 457 gcca agtttgaggt caacaaccca 481 caggtccagc gccaagcatt caatgagctc
atccgagtgg tccaccagct gttgccggaa 541 tccagcctca ggaagcggaa aaggagtcg 569 ataa

Amino acid sequence for Mouse IFNgamma

MNATHCILALQLFLMAVSGCYCHGTVIESLESLNNYFNSSGIDVEEKSLFLDIWRN
WQKDGDMKILQSQIISFYLRLFEVLKDNQAISNNISVIESHLITTFFSNSKAKKDAF
MSIAKFEVNNPQVQRQAFNELIRVVHQLLPESSLRKRKRSRC

Nucleotide sequence of the fused protein (BiPPB-IFN gamma)

tgt tct aga aac ctc atc gat tgt aag gga tcc gga ggt tgt tca cgt aat cta ata gat tgt tca
gcggccgca Interferon gamma sequence (NM_008337.3).

Amino acid sequence for BiPPB-IFNgamma

CSRNLIDCKGSGGCSRNLIDCSAAAMNATHCILALQLFLMAVSGCYCHGTVIESLE
SLNNYFNSSGIDVEEKSLFLDIWRNWQKDGDMKILQSQIISFYLRLFEVLKDNQAI
SNNISVIESHLITTFFSNSKAKKDAFMSIAKFEVNNPQVQRQAFNELIRVVHQLLPE
SSLRKRKRSRC

Italics denotes BiPPB; normal text denotes IFNgamma mimetic; Bold denotes linker or spacer. See also Figure 4.

### Mimetic Interferon gamma fused to BiPPB

Nucleotide sequence of the fused protein (BiPPB-IFN gamma mimetic)

tgt tct aga aac ctc atc gat tgt aag gga tcc gga ggt tgt tca cgt aat cta ata gat tgt tca
gcggccgca 457 gcca agtttgaggt caacaaccca caggtccagc gccaagcatt caatgagctc atccgagtgg
tccaccagct gttgccggaa tccagcctca ggaagcggaa aaggagtcg 569 ataa

Amino acid sequence for BiPPB-IFNgamma mimetic

CSRNLIDCKGSGGCSRNLIDCSAAA
AKFEVNNPQVQRQAFNELIRVVHQLLPESSLRKRKRSR*

Italics denotes BiPPB; normal text denotes IFNgamma mimetic; Bold denotes: linker or spacer. See also Figure 5.

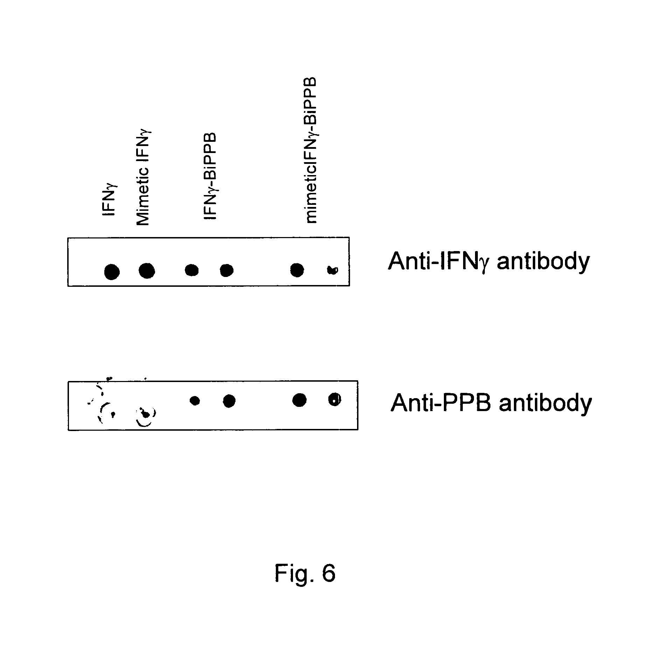

The nucleic acids were then transformed into BL21 cells (*E. coli)* for the expression using IPTG induction. The expressed protein was analysed by SDS-PAGE and Western blot analysis or Dot Blot analysis using anti-IFNγ antibody and/or anti-PPB antibody (see Figure 6). The expressed protein (with His Tag) was then purified under native conditions through Ni-NTA column chromatography. The tags were proteolytically cleaved and further purified using sepharose column chromatography.

**Example 2: Anti-fibrotic effects of cleaved and active mimetic IFN**γ: We evaluated the anti-fibrotic effects of cleaved IFNγ mimetic peptide in mouse NIH3T3 fibroblasts as assessed by immuno-cytochemistry (α-SMA staining). Briefly, 3T3 fibroblasts were seeded in 24 well plates at the density of 6 x 10⁴ cells/well, After 24 hrs, cells were starved in 0.5% FBS containing medium for overnight. Thereafter, cells were incubated in starvation medium with different compounds (PDGF 50 ng/ml, TGFβ 10 ng/ml, mimetic IFNγ 1µg/ml, 50ng/ml PDGF + 1µg/ml mimetic IFNγ and 10 ng/ml TGFβ + 1µg/ml mimetic IFNγ). After 48 hrs of incubation, cells were washed, fixed with ethanol:acetone (1:1) and stained for α-SMA (marker of activated fibroblasts).

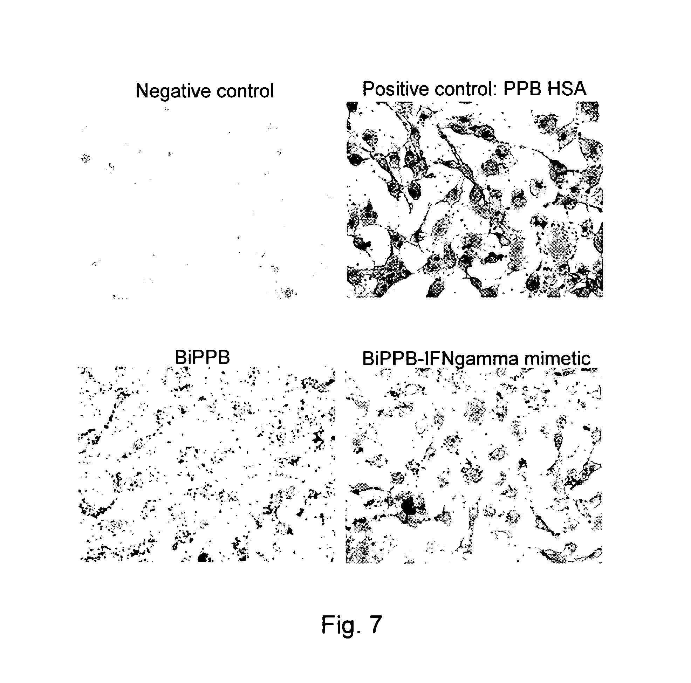

**Example 3: Binding study of BiPPB and mimetic IFNγ-BiPPB in human LX2 cells:** We determined the binding of BiPPB and mimetic-BiPPB in LX2 cells (human HSCs). Briefly, LX2 cells were plated in 48 well plates at the cell density of 3 x 10⁴ cells/well. After 24 hrs, cells were starved in medium (-FBS) for overnight. Then, cells were incubated with different compounds (BiPPB, PPB-HSA and BiPPB-Mimetic IFNγ) for 2 hrs at room temperature for binding. After binding, cells were extensively washed with PBS, fixed with ethanol:acetone (1:1) and stained for PPB. Results are presented in Figure 7.

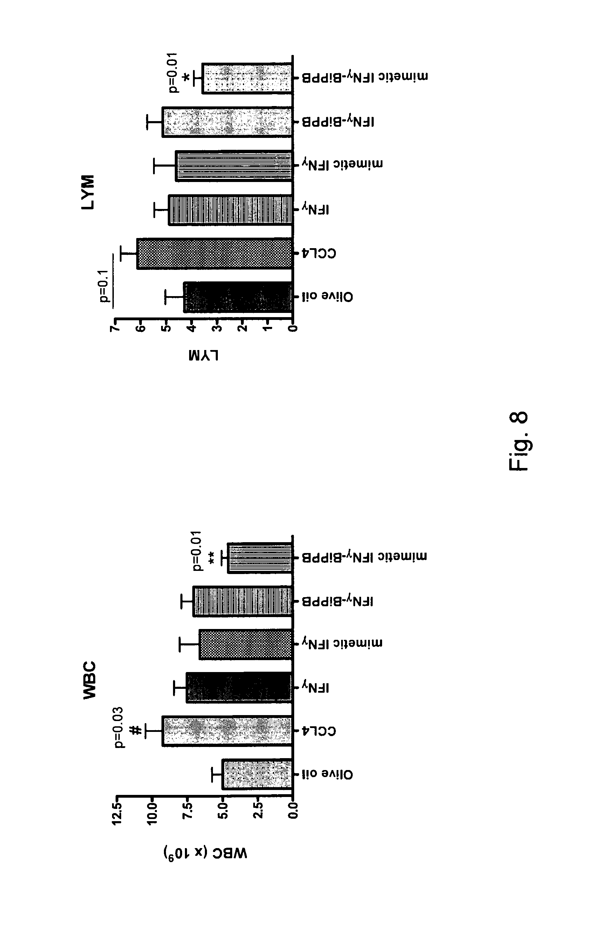

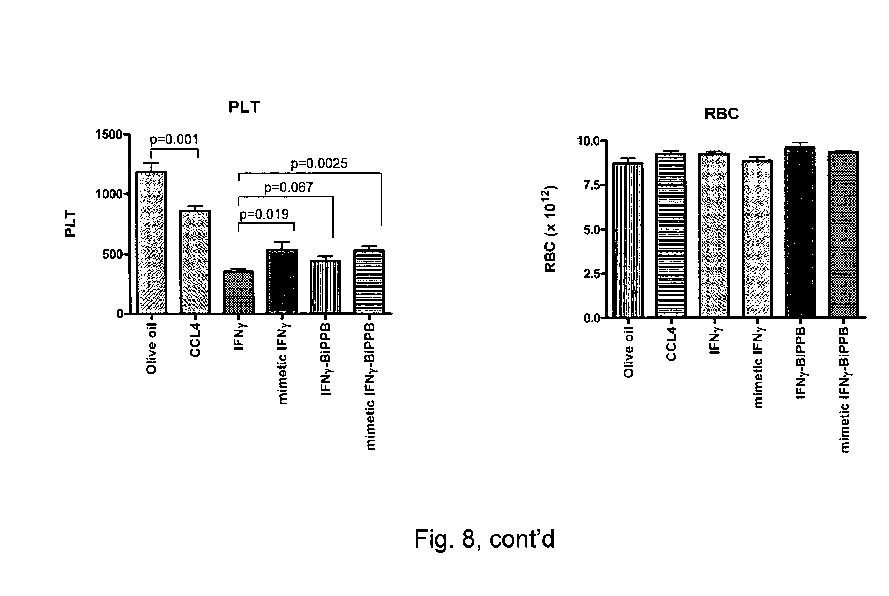

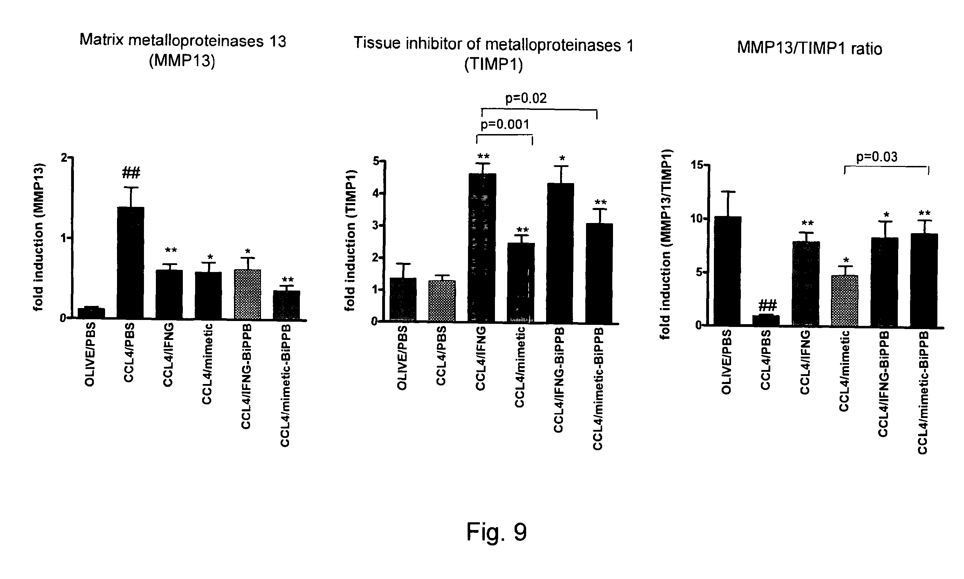

**Example 4: In-vivo effect study in acute CCl₄-induced liver injury in mice:** Recombinant IFNγ, mimetic IFNγ, recombinant fusion protein IFNγ-BiPPB and recombinant fusion protein mimetic IFNγ-BiPPB were tested for anti-fibrotic effects in acute CCL₄-induced liver injury mouse model. At day 1, the animals were given a single intra-peritoneal dose (lml/kg) of carbon tetrachloride (CCl₄) in olive oil or olive oil (controls n=6). After 24 hrs of CCl₄ injection, at day 2 and 3, animals were treated either with PBS (n=6), 50,000 U/mice of IFNγ (n=6), 50,000 U/mice of mimetic IFNγ (n=5), 50,000 U/mice of IFNγ-BiPPB (n=6), 50,000 U/mice of Mimetic IFNγ-BiPPB (n=6). Thereafter, at day 4, animals were sacrificed and blood counts were performed and anti-fibrotic effects (See Hemmann S, Graf J, Roderfeld M, Roeb. J Hepatol. 2007 May;46(5):955-75) were evaluated using quantitative PCR. Results are presented in Figures 8 and 9.

The data presented in Fig 8. demonstrate that mimetic-BiPPB is more effective than unmodified INFγ in attenuating the upregulation of white blood cell count (WBC) and lymphocyte count (lym) in whole blood associated with CCl₄-induced liver fibrosis (p<0.01). In contrast, the reduction in platelet count (PLT) associated with INFγ treatment (which is a well known adverse effect of INFγ) is less severe when animals receive mimetic-INFγ-BiPPB instead of unmodified INFγ (p< 0.0025).

The data presented in Figure 9 show that mimetic-INFγ-Bi-PPB is endowed with antifibrotic activity: it attenuates CCl₄-induced upregulation of matrix metalloproteinases (MMP13). The ratio MMP-13 versus Tissue Inhibitor of Metalloproteinases (TIPM1) is similar to that obtained with native INFγ, but better than mimetic INFγ (p<0.03), indicating that coupling of Bi-PPB to mimetic INFγ is beneficial. Collectively the data in Fig. 8 and 9 show that mimetic INFγ-BiPPB is more potent compared to native INFγ and has less side effects.

## Claims

1. An analog of interferon (IFN), wherein the moiety mediating binding to its natural receptor is at least functionally disrupted and wherein the analog comprises a signaling moiety capable of mediating intracellular IFN activity, said signaling moiety being provided at its N-terminus, optionally via a linker, with at least one targeting domain capable of binding to a cell surface receptor other than the IFN receptor.

2. Analog according to claim 1, being an interferon gamma (IFNγ) analog.

3. Analog according to claim 1 or 2, wherein the signaling moiety mediating intracellular activity comprises a polybasic nuclear localization signal (NLS) motif, preferably wherein the polybasic NLS motif comprises the amino acid sequence (R)KRXRS(R), wherein X is any amino acid residue, more preferably (R)KRXRS(R) wherein X is R, K, S or T.

4. Analog according to any one of the preceding claims, wherein the signaling moiety comprises a sequence selected from the group consisting of
a) the amino acid sequence
FEVNNPQVQRQAFNELIRVVHQLLPESSLRKRKRSR
b) the amino acid sequence
YSVTDLNVQRKAIHELIQVMAELSPAAKTGKRTRSQ
c) a stretch of at least 10, preferably at least 15, contiguous amino acids of the sequence of a) or b)
d) the consensus sequence
VxxxxVQRxAxxELIxVxxxLxPxxxxxKRxRS, wherein x is any amino acid
e) an amino acid sequence showing at least 70%, preferably at least 80%, more preferably at least 90% identity to a) or b) provided that the intracellular signaling activity is maintained; and
f) the amino acid sequence of a) or b) wherein at most 10, preferably at most 8, more preferably at most 5 amino acid residues are deleted, added or substituted, provided that the signaling activity is maintained.

5. Analog according to any one of the preceding claims, wherein the targeting domain can bind to a receptor that is specific for fibroblast and fibroblast-like cells, preferably myofobroblasts, portal fiborblasts, mesangial cells, interstitial fibroblasts, alveolar fibroblasts and/or stromal cells, preferably wherein the receptor is selected from the group consisting of the PDGF receptors, collagen type VI receptor, and cytokine receptors including TGFbeta receptor, TNFalpha receptor, Insulin growth factor receptors, VEGF receptors, chemokine receptors and IL1beta receptor.

6. Analog according to claim 5, wherein receptor is the PDGF receptor, preferably the PDGFβ receptor.

7. Analog according to any one of the preceding claims, wherein the targeting domain comprises at least one cyclic peptide portion, preferably wherein the targeting domain comprises at least one tandem repeat of a cyclic peptide portion, more preferably wherein the tandem repeat comprises two cyclic peptide portions, most preferably identical cyclic peptide portions, connected via a linker of 2 to 7 amino acids.

8. Analog according to any one of the preceding claims, wherein the targeting domain comprises at least the amino acid sequence RGD, KPT, SRN, NLI and/or LID.

9. Analog according to claim 8, wherein the targeting domain comprises the amino acid sequence CSRNLIDC-linker- CSRNLIDCS, wherein the linker is an amino acid sequence of 3 to 7, preferably 4 or 5, amino acid residues.

10. A conjugate comprising a compound of interest conjugated to a targeting domain, said targeting domain comprising the amino acid sequence X₁SRNLIDX₂-linker- X₃SRNLIDX₄, wherein the pair of X₁ and X₂ and the pair of X₃ and X₄ can form a (peptidic) bond such that a bicyclic structure is formed wherein the sequences SRNLID are part of a ring, and wherein the linker is an amino acid sequence of 2 to 7, preferably 3 to 5, amino acid residues.

11. Analog or conjugate according to any one of the preceding claims, provided with at least one non-antigenic polymer, preferably selected from the group consisting of polyethylene glycols and derivatives thereof.

12. An isolated nucleic acid sequence encoding a proteinaceous analog or conjugate according to any one of the preceding claims.

13. An expression vector comprising an isolated nucleic acid sequence according to claim 13.

14. A host cell comprising a nucleic acid sequence according to claim 13 or a vector according to claim 14, preferably wherein said host cell is a bacterial or mammalian host cell.

15. A pharmaceutical composition comprising an analog or conjugate according to any one of claims 1 to 12 and a pharmaceutically acceptable carrier.

16. Use of an analog or conjugate according to any one of claims 1 to 12 for the manufacture of a medicament for the therapeutic or prophylactic treatment of a disease selected from cancer, viral disease, fibrotic disease, sclerotic disease and chronic or acute inflammatory processes such as glomerulosclerosis, interstitial fibrosis, lung fibrosis, atherosclerosis, rheumatoid arthritis, Crohns disease, colitis ulcerosa, glomerulonephritis and sepsis, preferably wherein the disease is a liver disease, more preferably a chronic liver disease such as liver cirrhosis.
